(19) Europäisches Patentamt / European Patent Office / Office européen des brevets

(11) **EP 2 687 838 B1**

(12) **EUROPEAN PATENT SPECIFICATION**

(45) Date of publication and mention of the grant of the patent:
**11.09.2019  Bulletin 2019/37**

(51) Int Cl.:
*G01N 21/89* (2006.01)     *B65H 63/06* (2006.01)
*D01H 13/22* (2006.01)     *G01B 11/10* (2006.01)
*G01N 33/36* (2006.01)

(21) Application number: **13173808.0**

(22) Date of filing: **26.06.2013**

(54) **A DEVICE FOR MONITORING A QUALITY OF MOVING LINEAR TEXTILE MATERIAL AT AN OPERATING UNIT OF A TEXTILE MACHINE**

VORRICHTUNG ZUR ÜBERWACHUNG DER QUALITÄT EINES SICH BEWEGENDEN LINEAREN TEXTILMATERIALS AN EINER BETÄTIGUNGSEINHEIT EINER TEXTILMASCHINE

DISPOSITIF POUR SURVEILLER UNE QUALITÉ DE MATÉRIAU TEXTILE LINÉAIRE EN MOUVEMENT  AU NIVEAU D'UNE UNITÉ DE COMMANDE D'UNE MACHINE TEXTILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **20.07.2012  CZ 20120499**

(43) Date of publication of application:
**22.01.2014  Bulletin 2014/04**

(73) Proprietors:
  • **Rieter CZ s.r.o.**
    **562 01 Ústí nad Orlicí (CZ)**
  • **Ustav fyziky plazmatu AV CR, v.v.i.**
    **18200 Praha (CZ)**

(72) Inventors:
  • **BERAN, Zdenêk**
    **56134 Výprachtice (CZ)**
  • **KOUSALÍK, Pavel**
    **56203 Ústí nad Orlicí (CZ)**
  • **MELICH, Radek**
    **46822 Lísný (CZ)**

(74) Representative: **Musil, Dobroslav et al**
    **Zabrdovicka 801/11**
    **615 00 Brno (CZ)**

(56) References cited:
    WO-A2-2011/147385     CH-A5- 649 152
    US-A- 4 659 937

## Description

## Technical field

The technical solution relates to a device for monitoring a quality of moving linear textile material, particularly changes in its diameter and presence of foreign fibers and/or colour impurities in linear textile material, at an operating unit of a textile machine, comprising a radiation source and a linear image sensor, between which a space has been created for passage of textile material, whereby between the radiation source and the space for passage of textile material is arranged an optical member and further comprising sensors of radiation reflected from linear textile material.

## Background art

From the point of view of the quality of the yarn wound in a textile machine it is primarily the changes in yarn diameter and the presence of extraneous fibers and/or impurities in yarn that are monitored.

For measuring and detecting defects of yarn diameter, it is favourable to use sensors of the radiation emitted by a radiation source which monitor the shadow of the yarn passing between the radiation source and the sensor. For detecting extraneous fibers and yarn impurities, which generally appear to be colour foreign material, sensors are used, namely sensors of the radiation reflected from the yarn surface, on which the radiation emitted by the source falls.

A conventional solution is often based on using two individual sensors. However, this solution is complicated in itself, particularly due to the arrangement of the sensors in the path of yarn and the evaluation of yarn defects.

US 4,659,937 describes an apparatus for detecting surface defects and measuring the diameter of a moving object consisting of a wire. The apparatus comprises a radiation source, an optical system for focusing the optical rays as a laminar beam in a plane perpendicular to the longitudinal axis of the object, the optical system being situated between the source and the monitored object, and an optical detector system, situated in relation to the optical system on the opposite side of the object, for receiving the optical beams to generate a detector signal indicative of defects and the diameter. A first cylindrical lens is oriented with its longitudinal axis parallel to the source (if it comprises more that one diode) and perpendicular to the longitudinal axis of the object, whereby a second cylindrical lens is oriented perpendicular to the first lens. The portion of the laminar beam, that is not blocked by the object, is sensed by the optical detector system.

The first cylindrical lens is used to converge the optical rays to focus the image of the source on the object, while the second cylindrical lens is used to project a collimated beam in a plane perpendicular to the axis of the object. The combined action of the two cylindrical lenses produces a collimated beam perpendicular to the axis of the object. The combination of two independent cylindrical lenses poses high requirements on the precision and accuracy of the production of the resulting apparatus. If the apparatus was used for monitoring the diameter of yarn and for detecting the presence of foreign fibers, it would require the installation of of an independent device for detecting the presence of foreign fibers.

WO 2011/147385 describes a method and device for detecting the thickness and homogeneity of a linear formation, particularly textile fibers. The device comprises a light transmitter and a light receiver, and the formation to be monitored passes through the space between them. The transmitter is formed by a source of light and a diffuser which homogenizes the light, and the receiver comprises a focusing element in whose focus is arranged a shutter for eliminating non-parallel rays and behind the shutter there is an optical sensor which must be placed at the same distance from the shutter as the focusing element, or otherwise a collimation element must be arranged between the image sensor and the shutter. The device is complicated, poses high requirements on the precision of production and cannot be used for simultaneous detection of the yarn diameter and the presence of foreign fibers in yarn.

CZ PV 2009-634 describes a method of monitoring colour non-homogeneity of the yarn surface by means of monitoring and evaluating the radiation reflected from the yarn, which was emitted to the yarn by a radiation source, whereby the radiation reflected from the yarn is registered by a sensor of the reflected radiation in a common plane with sensing the absolute diameter of yarn by a linear digital optical sensor. Registering the absolute diameter of yarn and registering the radiation reflected from the yarn are mutually synchronized.

The device for carrrying out this method comprises a radiation source arranged next to the space for yarn passage and at least one sensor of the reflected radiation, whereby the device further comprises an evaluation device. Opposite the radiation source behind the space for yarn passage is arranged a linear digital optical sensor of absolute diameter of yarn, which is situated in a plane common with the radiation source and the sensor of the reflected radiation. The radiation source, the linear digital optical sensor, as well as the sensor of the reflected radiation, are each coupled with a source of control signals, these signals being mutually synchronized.

As a rule, the radiation source is made as a source with a relatively low power requirement, such as a LED diode. Considering the construction and properties of linear sensors of yarn diameter and sensors of the reflected radiation detecting colour impurities, requirements for radiation vary. When using a linear sensor it is advisable to irradiate intensely only the active surface of the linear sensor. It is effective to irradiate a short section of the moving yarn, i.e. to use a short, but relatively wide band of radiation, perpendicular to the axis of the yarn.

On the other hand, for detecting impurities of a different colour it is advisable to irradiate evenly a longer section of the yarn moving in front of a suitable background.

Nowadays there is no device that would meet this condition. Goal of the invention is to create at an operating unit of a textile machine a suitable device for monitoring the quality of moving linear textile material, particularly yarn.

## Principle of the invention

This goal has been achieved by a device according to appended claim 1. Advantageous embodiments are disclosed in appended claims 2 to 5.

In order to achieve optimum properties, the aspheric bi-cylindrical lens is made from polymethylmethacrylate.

Furthermore, It is advantageous if the radiation source is a green light-emitting diode (LED).

By using the aspheric bi-cylindrical lens it is possible to achieve a higher collimation of a beam of light rays, good-quality focusation (sharpness) of the light beam and increase in the lens numerical aperture.

By virtue of higher collimation the advantage of the device is its lower sensitivity to setting of the light intensity of the LED diode, lower sensitivity to variable yarn diameter in the measuring area, higher wear resistance of the LED diode and easier calibration of the device.

Good-quality focusation allows to increase energetic efficiency of the device, enables to use pulsed light, by which it is possible to eliminate a number of negative influences caused, for instance, by outer light, by "blurring" as a result of the period of sampling etc. Also, it is possible to create a more suitable shape of a light spot for measuring the reflected light.

Increasing numerical aperture enables to reduce the proportions of the device and also contributes to better effiency of light energy.

## Description of drawings

The device according to the invention is schematically shown in the enclosed drawings, where fig. 1 shows an arrangement of the device in the cross-section of a plane passing through the optical axis and being parallel with the direction of the movement of yarn with sensors of the radiation reflected from the yarn, fig. 2 illustrates an arrangement of the device in the cross-section of a plane passing through the optical axis and being perpendicular to the direction of the yarn without sensors of radiation reflected from the yarn, fig. 3 represents the function of an optical member in a plane passing through the optical axis and being perpendicular to the direction of the movement of yarn, fig. 4 represents the function of an optical member in a plane passing through the optical axis and being parallel with the direction of the movement of yarn, fig. 5 shows the course of radiation energy density in the section of the linear digital optical sensor for a standard spheric lens, fig. 6 shows the course of radiation energy density in the place of the linear digital optical sensor using an aspheric bi-cylindrical lens.

## Specific description

The device for monitoring the quality of moving linear textile material, particularly yarn, at an operating unit of a textile machine, comprises a source **1** of radiation, formed in the illustrated example of embodiment by a monochromatic LED diode **11** emiting green light, even though another radiation source can be also used. Opposite the source **1** of radiation is arranged a linear digital optical sensor **2**. Between the source **1** of radiation and the linear digital optical sensor **2** a space **30** has been created for the passage of linear textile material **3**, in the illustrated example of embodiment the passage of yarn. The space **30** for the passage of linear textile material **3** is from the side of the source **1** of radiation demarcated by a first transparent partition **41** and from the side of the linear digital optical sensor **2** is demarcated by a second transparent partition **42**. Between the second transparent partition **42** and the linear digital optical sensor **2** is arranged a slot aperture **5**, whose slot **51** is oriented parallel with the linear digital optical sensor **2**. The slot aperture **5** serves to supress the influence of the ambient stray light and in a case of a beam of rays larger than the height of the slot **51** the aperture will not let the peripheral rays through, but will either absorb them or reflect them.

Between the first transparent partition **41** and the source **1** of radiation is arranged an optical member **6**, which is formed by an aspheric bi-cylindrical lens, comprising a spheric cylindrical surface **61** having the axis of the cylinder parallel with the direction of the movement of the monitored textile material, and an aspheric cylindrical surface **62** having the axis of the cylinder perpendicular to the direction of the movement of the monitored textile material, whereby the spheric cylindrical surface **61** is directed to the radiation source.

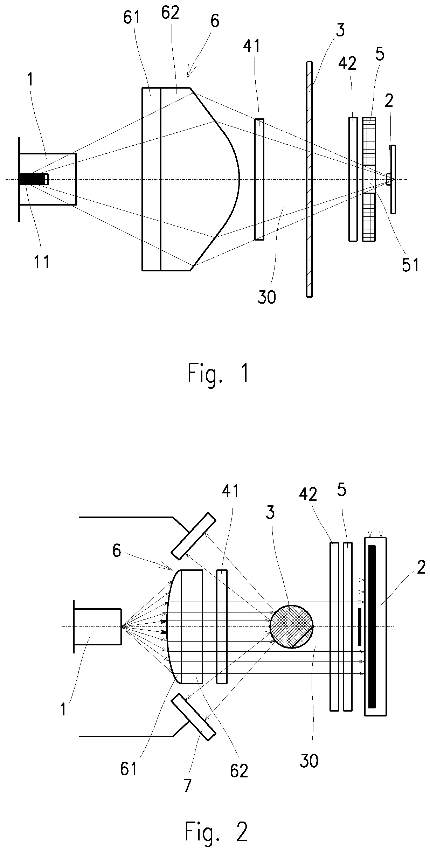

As is illustrated in fig. 2, outside the beam of rays of radiation emitted from the optical member **6** towards the monitored linear textile material **3**, there are next to the optical member **6** arranged sensors **7** of the radiation reflected from the surface of the monitored linear textile material **3**.

As mentioned before, for monitoring the diameter of linear textile material **3** it is advantageous to irradiate with great intensity only the active surface of the linear digital optical sensor **2**. That means using a vertically narrow beam of rays of radiation, which irradiates the monitored linear textile material **3** and which is focused into the slot of the slot aperture **5**. On the other hand, for monitoring the presence of extraneous fibers and impurities in linear textile material **3** it is advisable to apply uniform radiation of the slot aperture **5** and a longer section of linear textile material **3**, which passes in front of a suitably chosen background formed by a slot aperture **5.** That represents a less intense beam of radiation rays, which irradiates uniformly the reflective surface of the aperture **5** and sufficiently illuminates the surface of the monitored linear textile material **3.** The integration of both these principles into one optical plane is provided by using an aspheric bi-cylindrical lens, which is formed by two interfaces. The first interface in the direction of radiation is the interface air - optical material. The second interface is the interface optical material - air.

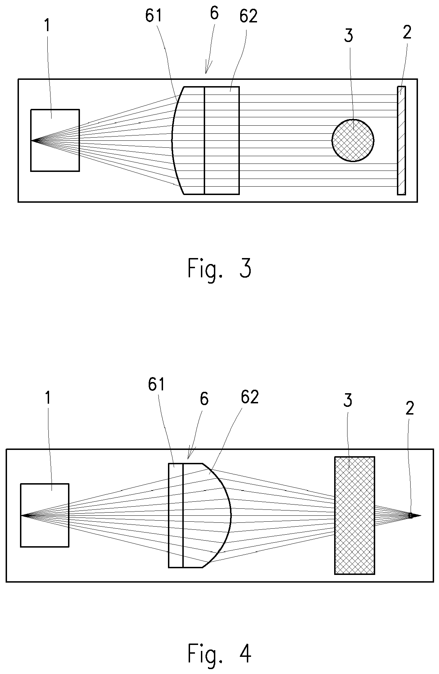

The first interface, which is schematically represented in fig. 3, is a spheric cylindrical surface **61** having the axis of the cylinder parallel with the direction of the movement of the monitored linear textile material **3.** The spheric cylindrical surface is determined by the equation:

$$z = \frac{cx^2}{1 + \sqrt{1 - (1+k)c^2x^2}}$$

where:

c represents the curvature of the respective surface, or, the reversed value of radius,
the coefficient k represents the conical coefficient of the respective surface, defining the type of conic section (for k = 0 it relates to sphere, for k > -1 ellipsis, for k = -1 paraboloid, for k <-1 hyberboloid)
x represents the distance of the lens surface from its axis.

The function of this spheric cylindrical surface **61** in the optical system is collimation, i.e. creating a beam of parallel radiation rays emerging from the object plane, in which the source **1** of radiation is located.

The second interface, which is schematically shown in fig. 4, is an aspheric cylindrical surface **62** having the axis of the cylinder perpendicular to the direction of the movement of the monitored linear textile material **3.**

The aspheric cylindrical surface is determined by the equation:

$$z = \frac{cy^2}{1 + \sqrt{1 - (1+k)c^2y^2}} + A_4 y^4$$

where:

$A_4$ (or others $A_6$, $A_8$, etc. - not shown) represents other aspheric coefficients specified in polynomial development
y represents the distance of the surface of the lens from it axis.

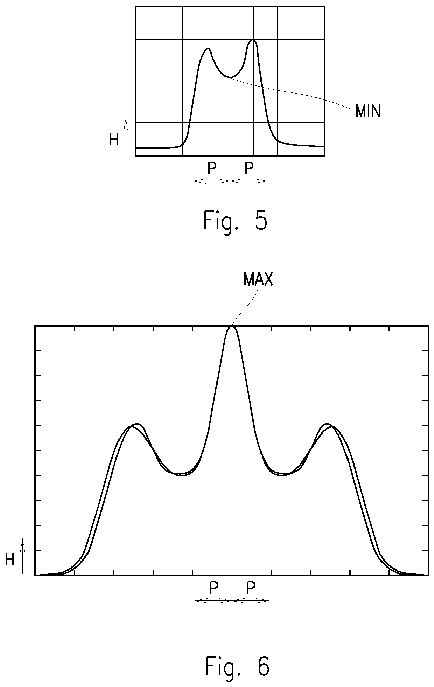

The function of this aspheric cylindrical surface **62** is focusing the beam of rays on the active surface of the linear digital optical sensor **2,** as well as increasing numerical aperture, which means the ability of the lens to absorb more light. This is caused by the fact that the aspheric cylindrical surface **62** allows to locate the source **1** of radiation closer to the optical member **6,** and thus to increase the amount of the transmitted radiation in the beam of rays focused on the active surface of the linear digital optical sensor **2** and at the same time to keep sufficient quality of projection. This part of the beam of rays passes through the slot **51** of the aperture **5** and is focused directly on the narrow band covering the surface of the linear digital optical sensor **2,** which is, as a result, intensely irradiated. Thus the shadow representing the thickness of the monitored linear textile material **3** is precisely determined, whereby it does not affect the impassable reflective part of the aperture **5.** The remaining part of the beam of rays irradiates uniformly, but considerably less intensely, a greater length of the monitored linear textile material **3.** That contributes to increased sensitivity of detection of extraneous fibers and colour impurities in the monitored linear textile material **3.** The corresponding course of the density **H** of radiation energy (fig. 6) in the section of the linear digital optical sensor **2** has actually its local maximum MAX in the optical axis of the optical member **6,** in contrast to the standard spheric lens (fig. 5), where there is the local minimum in this section and its local maximum values are distributed on its sides.

In order to extend the radiation spot on the monitored linear textile material **3** the aspheric cylindrical surface **62** can be adjusted by spherical aberration, which arises as a result of deviation from the ideal geometrical shape. This leads to the extention of the area of the irradiated part of the surface of the the monitored linear textile material and permits smoother evaluation of extraneous fibers and colour impurities in the linear textile material and at the same time allows increased tolerance of positioning individual members of the optical system.

The above-described optical member **6** enables to use sources of radiation of lower intensity, for example a green light-emitting LED diode.

In a preferable embodiment the lens of the optical member **6** is made from polymethylmethacrylate. However, other suitable materials can also be used, whereby it is necessary to adapt the shape of the profile of the cylindrical surfaces to the particular material.

### List of references

| | |
|---|---|
| 1 | radiation source |
| 11 | LED diode |
| 2 | linear digital optical sensor |
| 3 | linear textile material |
| 30 | space for linear textile material passage |
| 41 | first transparent partition |
| 42 | second transparent partition |
| 5 | slot aperture |

51   slot
6    optical member
61   spheric cylindrical surface
62   aspheric cylindrical surface
7    sensor of reflected radiation

**Claims**

1.  A device for monitoring the quality of moving linear textile material (3), particularly changes in its diameter and the presence of extraneous fibers and/or colour impurities in the linear textile material, at an operating unit of a textile machine, comprising a source (1) of radiation and a linear digital optical sensor (2), between which a space (30) has been created for passage of linear textile material (3), whereby between the radiation source (1) and the space (30) for passage of linear textile material (3) is arranged an optical member (6) and further comprising sensors (7) of radiation reflected from the linear textile material (3), **characterized in that** the optical member (6) is formed by an aspheric bi-cylindrical lens, comprising a spheric cylindrical surface (61) directed towards the radiation source and having the axis of the cylinder parallel with the direction of the movement of the monitored textile material (3) for the collimation of a beam of rays, and an aspheric cylindrical surface (62) having the axis of the cylinder perpendicular to the direction of the movement of the monitored textile material (3) for focusing the beam of rays on the active surface of the linear digital optical sensor, for increasing the numerical aperture of the optical member (6), as well as for uniform irradiation of a greater length of the monitored linear textile material (3) and of the reflecting surface of an aperture slot (5) so as to increase the sensitivity of detection of extraneous fibers and/or colour impurities in the monitored linear textile material (3).

2.  A device according to Claim 1, **characterized in that** the aspheric cylindrical surface (62) is shape-modified for the purpose of achieving spherical aberration for extending the radiation spot on the monitored linear textile material.

3.  A device according to any of the preceding Claims, **characterized in that** the aspheric bi-cylindrical lens is made from optically transparent material enabling refraction of light rays.

4.  A device according to any of the preceding Claims, **characterized in that** the radiation source (1) is a LED diode.

5.  A device according to Claim 4, **characterized in that** the LED diode emits green light.

**Patentansprüche**

1.  Einrichtung zur Ermittlung der Qualität des sich bewegenden linearen Textilmaterials (3), insbesondere der Änderungen von seinem Durchmesser und der Anwesenheit von Fremdfasern und/oder Farbbeimischungen im linearen Textilmaterial, auf der Arbeitsstelle der Textilmaschine, die eine Strahlungsquelle (1) und einen digitalen Zeilenbildsensor (2) aufweist, zwischen denen ein Raum (30) zum Durchgang des linearen Textilmaterials (3) gebildet wird, wobei zwischen der Strahlungsquelle (1) und dem Raum (30) zum Durchgang des linearen Textilmaterials (3) ein optisches Glied (6) angeordnet ist, und die weiter Sensoren (7) der Strahlung aufweist, die von dem linearen Textilmaterial (3) reflektiert wird, **dadurch gekennzeichnet, dass** das optische Glied (6) durch eine asphärische bizylindrische Linse, die eine sphärische zylindrische Fläche (61) aufweist, die zur Strahlungsquelle mit der Zylinderachse gerichtet ist, die mit der Bewegungsrichtung des zu verfolgenden Textilmaterials (3) zur Kollimation des Strahlbündels parallel laufend ist, und eine asphärische zylindrische Fläche (62) mit der Zylinderachse gebildet wird, die zur Bewegungsrichtung des zu verfolgenden Textilmaterials (3) zur Fokussierung des Strahlbündels auf die aktive Fläche des digitalen optischen Zeilensensors, Vergrößerung der nummerischen Apertur des optischen Gliedes (6) und zur gleichmäßigen Bestrahlung der größeren Länge des zu verfolgenden linearen Textilmaterials (3) und der Reflexionsfläche der Blende (5) zur Erhöhung der Empfindlichkeit der Detektion von Fremdfasern und/oder Farbbeimischungen in dem zu verfolgenden linearen Textilmaterial (3) senkrecht ist.

2.  Einrichtung nach dem Anspruch 1, **dadurch gekennzeichnet, dass** die asphärische zylindrische Fläche (62) zur Erreichung der sphärischen Aberration zur Erweiterung der Strahlungsspur auf dem zu verfolgenden linearen Textilmaterial formmodifiziert wird.

3.  Einrichtung nach einem der vorangehenden Ansprüche, **dadurch gekennzeichnet, dass** die asphärische bizylindrische Linse aus einem optisch transparenten Material hergestellt ist, das die Brechung von Lichtstrahlen ermöglicht.

4.  Einrichtung nach einem der vorangehenden Ansprüche, **dadurch gekennzeichnet, dass** die Strahlungsquelle (1) eine LED-Diode ist.

5.  Einrichtung nach dem Anspruch 4, **dadurch gekennzeichnet, dass** die LED-Diode ein grünes Licht aufweist.

**Revendications**

1. Dispositif pour la détection de la qualité d'un matériau textile linéaire en mouvement (3), en particulier de la modification de son diamètre et de la présence des fibres étrangères et/ou d'impuretés de couleur dans un matériau textile linéaire, sur un poste de travail d'une machine textile, comprenant une source (1) de rayonnement et un capteur d'image numérique linéaire (2) entre lesquels un espace (30) est formé pour le passage du matériau textile linéaire (3), tandis que un élément optique (6) est disposé entre la source de rayonnement (1) et le passage de matériau textile linéaire (30) et il comprend en outre des capteurs de rayonnement (7) réfléchis par le matériau textile linéaire (3), **caractérisé en ce que** l'élément optique (6) est formé par une lentille bicylindrique asphérique comprenant une surface cylindrique sphérique (61) dirigée vers une source de radiation avec un axe cylindrique parallèle au sens de déplacement du matériau textile observé (3) pour la collimation du faisceau des rayons et la surface cylindrique asphérique (62) avec l'axe cylindrique perpendiculaire à la direction de déplacement du matériau textile observé (3) pour la focalisation du faisceau des rayons sur la surface active du capteur optique numérique en ligne, pour l'agrandissement de l'ouverture numérique de l'élément optique (6) et pour l'irradiation uniforme de la plus grande longueur du matériau textile linéaire observé (3) et les surfaces réfléchissantes de l'ouverture (5) pour l'augmentation de la sensibilité de la détection des fibres étrangères et/ou des impuretés de couleur du matériau textile linéaire observé (3).

2. Dispositif selon la revendication 1, **caractérisé en ce que** la surface cylindrique asphérique (62) modifie sa forme pour réaliser une aberration sphérique afin d'étendre la plus grande trace du rayonnement sur le matériau textile linéaire observé.

3. Dispositif selon l'une quelconque des revendications précédentes, **caractérisé en ce que** la lentille bicylindrique asphérique est réalisée en un matériau optiquement transparent permettant la réfraction des rayons lumineux.

4. Dispositif selon l'une quelconque des revendications précédentes, **caractérisé en ce que** la source de rayonnement (1) est une diode LED.

5. Dispositif selon la revendication 4, **caractérisé en ce que** la diode LED a une lumière verte.

Fig. 1

Fig. 2

Fig. 3

Fig. 4

Fig. 5

Fig. 6

**REFERENCES CITED IN THE DESCRIPTION**

**Patent documents cited in the description**

- US 4659937 A
- WO 2011147385 A
- CZ PV2009634